# EUROPEAN PATENT SPECIFICATION

(11) **EP 3 039 403 B1**
(45) Date of publication and mention of the grant of the patent: **20.06.2018**
(21) Application number: 14765972.6
(22) Date of filing: 26.08.2014
(51) Int. Cl.: G01N 15/06, C02F 1/44, G01N 33/18, G01N 21/64, C02F 1/00, G01N 31/22, G01N 21/77

(54) **METHOD FOR DETECTING TRANSPARENT EXOPOLYMER PARTICLES IN A WATER SAMPLE**
VERFAHREN ZUR ERKENNUNG TRANSPARENTER EXOPOLYMERTEILCHEN IN EINER WASSERPROBE
PROCÉDÉ DE DÉTECTION DE PARTICULES D'EXOPOLYMÈRES TRANSPARENTES DANS UN ÉCHANTILLON D'EAU

(30) Priority: 26.08.2013 FI 20135857
(43) Date of publication of application: 06.07.2016
(73) Proprietor: Kemira Oyj, 00180 Helsinki (FI)
(72) Inventor: SAARI, Eija, FI-02230 Espoo (FI); KARISALMI, Kaisa, FI-00510 Helsinki (FI); HESAMPOUR, Mehrdad, FI-02660 Espoo (FI); VIRKKI, Heli, FI-00140 Helsinki (FI); NIEMELÄ, Miia, FI-00700 Helsinki (FI); KORTE, Eija, FI-02260 Espoo (FI)
(74) Representative: Berggren Oy, Turku
(86) International application number: PCT/FI2014/050648
(87) International publication number: WO 2015/028711

(56) References cited:
- EP-A1- 2 463 241
- WO-A2-2004/009497
- US-A1- 2007 036 682
- VILLACORTE L O ET AL: "The fate of Transparent Exopolymer Particles (TEP) in integrated membrane systems: Removal through pre-treatment processes and deposition on reverse osmosis membranes", WATER RESEARCH, ELSEVIER, AMSTERDAM, NL, vol. 43, no. 20, 28 August 2009 (2009-08-28), pages 5039-5052, XP026790159, ISSN: 0043-1354 [retrieved on 2009-08-28] cited in the application
- VILLACORTE L O ET AL: "Characterisation of transparent exopolymer particles (TEP) produced during algal bloom: a membrane treatment perspective", DESALINATION AND WATER TREATMENT : SCIENCE AND ENGINEERING ; DWT, TAYLOR & FRANCIS, UK, vol. 51, no. 4-6, 1 January 2013 (2013-01-01), pages 1021-1033, XP008172711, ISSN: 1944-3994, DOI: 10.1080/19443994.2012.699359 [retrieved on 2012-07-24]
- Sven Uthicke ET AL: "Fluorescent lectin assay to quantify particulate marine polysaccharides on 96-well filtration plates", Limnol. Oceanogr.: Methods, vol. 7 13 April 2009 (2009-04-13), pages 449-458, XP055355423, Retrieved from the Internet: URL:http://m.aslo.net/lomethods/free/2009/ 0449.pdf [retrieved on 2017-03-16]

## Description

The invention relates to a method for detecting transparent exopolymer particles in a water sample according to preambles of the enclosed independent claims.

Transparent exopolymer particles, TEP, may be present in aqueous environments, both in natural waters and in industrial process waters or wastewaters. They are transparent biopolymer particles mainly made up of acidic polysaccharides from phytoplanktons and bacterioplanktons, and may have a form of deformable strings, disks or films up to several 100 µm long. TEP are very sticky, flexible and surface reactive, and they behave like gels and increase viscosity. They may coalesce in order to form larger gels and porous networks, and they easily cause biofouling, e.g. fouling of membranes in desalination processes, especially during algal blooms.

Hitherto the significance of TEP to various process problems in water-rich industries has been largely overlooked, because they have been extremely hard to detect and quantify. TEP are transparent, which means that they cannot be directly detected. One method for detecting TEP is described by U. Passow and A.L. Alldredge in Limnol. Oceanogr. 40(7), 1995, 1326 - 1335. In the described method TEP is stained with cationic dye Alcian Blue and the amount of dye complexed with TEP is colorimetrically determined. The detection method comprises a plurality of different steps: filtering, staining, washing, soaking and colorimetric determination. It is clear that the method is not suitable for industrial use, where rapid response is required. Furthermore, the interaction between the dye and TEP is based on ionic interaction and is non-selective. For example, possible anionic polymers or anionic impurities disturb the determination of TEP.

US 2007/0036682 discloses a microoptic glucose detector. It provides a fully-integrated microfluidic system for detection of the fluorescence which comprises a light source, a self-assembled monolayer of a compound coated on a gold surface that fluoresces in the presence of a saccharide, a light processor and a detector.

WO 2004/009497 discloses a method for monitoring and/or controlling biofouling in membrane separation systems. The method utilizes measurable amounts of fluorogenic agent(s) added to a feed stream to monitor and/or control the level of microbial growth during membrane separation and the purification of the feed stream during membrane separation.

S. Utchike et al., Limnol. Oceanogr.: Methods 7, 2009, 449-458, discloses fluorescent lectin assay to quantify particulate marine polysaccharides on 96-well filtration plates. The developed method quantifies more broadly particulate polysaccharides and complements Alcian blue quantification of transparent exopolymer particles, which are subset of the ocean's pool of particulate polysaccharides. The method uses fluorescently labelled lectin concanavalin A that binds polysaccharides that contain glucose and mannose.

An object of the present invention is to minimise or even eliminate the disadvantages existing in the prior art.

An object of the invention is also to provide a method with which transparent exopolymer particles may be rapidly and reliably detected.

Another object of the invention is to provide a method with which the amount of transparent exopolymer particles may be easily monitored.

These objects are attained with the invention having the characteristics presented below in the characterising parts of the independent claims.

A typical method according to the present invention for detecting transparent exopolymer particles in a water sample, comprises
- obtaining a water sample,
- introducing a fluorochromatic reagent to the water sample, the fluorochromatic reagent being specific to vicinal hydroxide groups of transparent exopolymer particles, whereby the fluorescence signal of the reagent changes when it comes into contact with transparent exopolymer particles, i.e. TEP,
- detecting the fluorescence signal from the water sample and determining the TEP level of the sample.

Now it has been surprisingly found out that a fast and reliable analysis of the amount of transparent exopolymer particles can be obtained if they are allowed to interact with a specific fluorochromatic reagent, and then the fluorescence signal is detected. This enables creation of rapid method, which can be used for industrial analysis of TEP in different natural or process waters. The invention makes it possible to improve the operational efficiency in water-intensive processes, where TEP concentration may vary significantly, for example, due to seasonal variations. The present method provides also possibility to obtain a greater insight in the water treatment process.

In the present invention a fluorochromatic reagent is introduced to the water sample. In this context the term "fluorochromatic" means a compound that fluoresces. The reagent is added in known, predetermined amount, which makes the obtained signals comparable with each other. A person skilled in the art may, without undue burden, determine suitable reagent amounts for each system.

According to one embodiment of the invention the fluorochromatic reagent is specific to vicinal hydroxide groups. In other words, the fluorochromatic reagent is specific to transparent exopolymer particles comprising at least two hydroxide groups attached to adjacent atoms. Thus, the reagent is selective to e.g. diols and triols. The fluorescence signal of the reagent changes when it comes into contact with vicinal hydroxide groups of transparent exopolymer particles.

According to one embodiment of the invention the fluorochromatic reagent is a boronic acid derivative, for example a phenylboronic acid derivative, such as 3-(dansylamino)phenylboronic acid (DAPB), 3,4,5-trifluorophenylboronic acid, 2-fluoro-5-nitrophenylboronic acid, 2-methoxyphenylboronic acid, N-benzyl-3-pyridiniumphenylboronic acid, o-dimethylaminomethylphenylboronic acid, 3-chloro-4-fluorophenylboronic acid or 4-bromophenylboronic acid. Further, the fluorochromatic reagent may be a boronic acid derivative, such as 8-quinolineboronic acid (8-QBA); 5-quinolineboronic acid (5-QBA); 6-(dimethylamino)-naphthalene-2-boronic acid (6-DMANBA); or phenoxathiin-4-boronic acid (4-POBA). The response which signals an interaction between TEP and boronic acid derivative is communicated by changes in fluorescence intensity either through chelation enhanced quenching or chelation enhanced fluorescence.

According to one preferred embodiment of the invention the fluorochromatic reagent is 3-(dansylamino)phenylboronic acid (DAPB). 3-(dansylamino)-phenylboronic acid is cheap and easily available, whereby it is suitable for industrial purposes. DAPB interacts with vicinal diols and certain amino alcohols to form cyclic complexes that a have a fluorescence intensity and peak emission dependent on the environment of the fluorophore.

According to one embodiment of the invention the fluorescence signal of the free non-interacted fluorochromatic reagent is detected, for example when 3-(dansylamino)phenylboronic acid (DAPB) is used as fluorochromatic reagent. This means that the detected signal decreases with increasing TEP concentration. The amount of the fluorochromatic reagent, which is used, is sufficient when a fluorescence signal that is typical for the unreacted reagent is still obtainable. When the detected signal is smaller than a predetermined threshold signal value, it is a clear indication that the TEP concentration in the water sample has exceeded the permitted level.

According to another embodiment of the invention the fluorescence signal of the interacted fluorochromatic reagent is detected, for example when 8-quinolineboronic acid (8-QBA), 5-quinolineboronic acid (5-QBA) or 6-(dimethylamino)-naphthalene-2-boronic acid (6-DMANBA) is used as fluorochromatic reagent. This means that the detected signal increases with increasing TEP concentration. When the detected signal is stronger than a predetermined threshold signal value, it is a clear indication that the TEP concentration in the water sample has exceeded the permitted level.

According to one embodiment of the invention the water sample to be analysed for TEP is obtained from a water treatment process, and the feed of one or several process chemicals is adjusted and/or selected on basis of the detected fluorescence signal. For example, if the detected signal indicates that the TEP concentration exceeds the permitted level, it is possible to start feeding new chemicals to the process for reducing the concentration of TEP. Alternatively or simultaneously, the dose of constantly fed process chemicals may be changed for reducing the TEP concentration. Examples of such chemicals, feed of which may be adjusted and/or selected on basis of the detected signal, are coagulants and flocculants. When the feed and/or dosing of treatment chemicals is done based on the detected signal, the chemical costs may be reduced, as overfeeding may be avoided.

According to one embodiment of the invention the pH of the water sample is adjusted to a constant value before introduction of the fluorochromatic reagent. It has been observed that at least for some reagents the detected fluorescence signal may also be dependent on pH of the sample, in which case it is preferred to adjust the pH to a constant value in order to eliminate the pH dependency of the detected signal. pH adjustment may be performed by introduction of a suitable buffering agent, e.g. boric acid/potassium chloride/sodium hydroxide, or by introduction of a suitable strong base, such as NaOH, or acid, to the water sample. In such processes where the pH is constant, and very close to the value at which the fluorescence intensity maximum of the reagent is obtained, no pH adjustment step is necessary. A person skilled in the art is able determine the optimal pH range for used fluorochromatic reagent and for each sample system by using known methods.

When a phenylboronic acid derivative, such as 3-(dansylamino)phenylboronic acid (DAPB) is used as fluorochromatic reagent pH of the sample may be adjusted to a level > 7, more preferably > 8, in order to optimise the intensity of the fluorescence signal. pH of the water sample may be adjusted to the range of 7 - 10, more typically 8.5 - 9.5.

When 8-quinolineboronic acid (8-QBA) is used as fluorochromatic reagent pH of the sample may be adjusted to pH 4 - 10, more preferably pH 4.5 - 7.5, in order to optimise the intensity of the fluorescence signal.

When 5-quinolineboronic acid (5-QBA) is used as fluorochromatic reagent pH of the sample may be adjusted to pH > 4, more preferably pH > 7.5, in order to optimise the intensity of the fluorescence signal. pH of the water sample may be adjusted to the range of 7 - 10, more typically 8.5 - 9.5.

When phenoxathiin-4-boronic acid (4-POBA) is used as fluorochromatic reagent pH of the sample may be adjusted to pH 2 - 7, more preferably pH 2 - 4, in order to optimise the intensity of the fluorescence signal.

Preferably, the water sample is obtained or taken as a side stream from an aqueous process stream, for example in a water treatment process comprising a pre-treatment unit and a reverse osmosis unit. The water sample may be taken before or after the pre-treatment unit. A pH adjustment agent, such as buffering agent, may be introduced to the water sample side stream, and preferably, after reaching the desired pH level, the fluorochromatic reagent may be introduced continuously or at pre-determined intervals to the water sample side stream, and the fluorescence signal may be detected, respectively, continuously or at pre-determined intervals. The detected signal may optionally be filtered, if necessary, and/or it may be mathematically analysed. The TEP level in the process stream is determined by comparing the detected signal to predetermined reference signal(s).

The TEP level in the process stream may be determined by comparing the detected signal to predetermined reference signal(s). For example, when quenching of the signal of the fluorochromatic reagent in presence of TEP is detected, a fluorescence signal of reagent in ultrapure water may be used as a reference signal, whereby a maximum signal without any TEP is obtained. Alternatively a relative value for the TEP amount can be calculated by comparing the detected signal to the signal of ultrapure water. The signal of ultrapure water is given the value 100 and if there is TEP present in the sample the signal quenching is scaled accordingly.

Since natural waters may be a turbid media, the predominance of light scattering may become significant and distort the fluorescence emission spectrum. In that case it is possible, for example, to correct the detected fluorescence signal by considering the Raman scattering.

According to one embodiment of the invention the fluorescence signal is detected by using spectrophotometry or spectrofluorometry, for example by using cuvette, flow-through cuvette or probe. All these detection methods are known as such for a person skilled in the art.

The method according to the present invention is suitable for all water-intensive processes, where TEP may be present. Examples of such processes are different water purification processes, such as desalination, pre-treatment and water intensive manufacturing processes, such as pulping and paper making. Typically such processes in which the invention is useful include at least one microfiltration, ultrafiltration, nanofiltration and/or reverse osmosis step(s).

The method described herein may be used to detect or determine extracellular polysaccharides (EPS).

### EXPERIMENTAL

Brackish water was treated with different doses of ferric chloride (trade name: PIX-111, Kemira Oyj) at pH 5.5. 750 µl samples of treated water and untreated reference water were taken and 250 µl of buffer solution boric acid/potassium chloride/sodium hydroxide, pH 9, was added.

2.00·10⁻⁴ M 3-(dansylamino)phenylboronic acid (DAPB) reagent was made by dissolving it to 1 ml dimethylsulfoxide and diluting to 50 ml with water. 20 µl of this DAPB solution was added to each buffered water sample and the fluorescence of the samples was measured using spectrofluorometer, excitation wavelength was 325 nm and the emission intensities between 470 - 600 nm were integrated. The emission sum was then divided by the intensity of the Raman scattering peak at 650 nm. The result was then compared to the reference sample of ultrapure water. The unit is the decrease (%) of the relative intensity of the sample vs. the ultrapure water (reference).

For a comparison, transparent exopolymer particles were also determined from the same water samples using Villacorte's method (Villacorte LO, Kennedy MD, Amy GL, Schippers JC (2009): The fate of transparent exopolymer particles (TEP) in integrated membrane systems: Removal through pre-treatment processes and deposition on reverse osmosis membranes. Water Research 43: 5039-5052) and the results were compared, see Table 1. From the results can be seen that the relative results of the method according to the invention correlate with the results achieved with Villacorte's method. The measurement unit in Villacorte's method is mg Xanthan equivalent per liter using an arbitrary calibration factor of 0.114 mgXeq.

**Table 1. Relative results of TEP measurements.**

| **Treatment\method** | **TEP total (Villacorte), AVG** | **Relative TEP (DAPB), AVG** |
|---|---|---|
| Untreated | 1.60 | 75.5 |
| 25 ppm PIX-111 | 1.02 | 65.4 |
| 75 ppm PIX-111 | 0.73 | 31.8 |

## Claims

1. Method for detecting transparent exopolymer particles in a water sample, the method comprising
- obtaining a water sample,
- introducing a fluorochromatic reagent to the water sample, the fluorochromatic reagent being specific to vicinal hydroxide groups of transparent exopolymer particles, whereby the fluorescence signal of the reagent changes when it comes into contact with transparent exopolymer particles,
- detecting the fluorescence signal from the water sample and determining the transparent exopolymer particle level of the sample.

2. Method according to claim 1, **characterised in that** the fluorochromatic reagent is boronic acid derivative, such as 8-quinolineboronic acid; 5-quinolineboronic acid; 6-(dimethylamino)-naphthalene-2-boronic acid; or phenoxathiin-4-boronic acid.

3. Method according to claim 1, **characterised in that** the fluorochromatic reagent is a phenylboronic acid derivative, such as 3-(dansylamino)phenylboronic acid , 3,4,5-trifluorophenylboronic acid, 2-fluoro-5-nitrophenylboronic acid, 2-methoxyphenylboronic acid, N-benzyl-3-pyridiniumphenylboronic acid, o-dimethylaminomethylphenylboronic acid, 3-chloro-4-fluorophenylboronic acid or 4-bromophenylboronic acid, preferably 3-(dansylamino)phenylboronic acid.

4. Method according to claim 1, **characterised in** obtaining the water sample from a water treatment process, and adjusting and/or selecting the feed of one or several process chemicals on basis of the detected fluorescence signal.

5. Method according to any of preceding claims 1 - 4, **characterised in** adjusting the pH of the water sample to a constant value before introduction of the fluorochromatic reagent.

6. Method according to claim 5, **characterised in that** the fluorochromatic reagent is a phenylboronic acid derivative, such as 3-(dansylamino)phenylboronic acid, and the pH of the water sample is adjusted to a level > 7, more preferably > 8.

7. Method according to claim 5, **characterised in that** the fluorochromatic reagent is 8-quinolineboronic acid and the pH of the water sample is adjusted to pH 4 - 10, more preferably pH 4.5 - 7.5.

8. Method according to claim 5, **characterised in that** the fluorochromatic reagent is 5-quinolineboronic acid and the pH of the water sample is adjusted to pH > 4, more preferably pH > 7.5.

9. Method according to claim 5, **characterised in that** the fluorochromatic reagent is phenoxathiin-4-boronic acid the pH of the water sample is adjusted to pH 2 - 7, more preferably pH 2 - 4.

10. Method according to any of preceding claims 1 - 9, **characterised in**
- obtaining the water sample as a side stream from a aqueous process stream,
- introducing the fluorochromatic reagent to the side stream and detecting the fluorescence signal,
- optionally filtering the signal,
- determining the transparent exopolymer particle level in the process stream by comparing the detected signal to predetermined reference signal(s).

11. Method according to claim 1, **characterised in** detecting the fluorescence signal of the free non-interacted fluorochromatic reagent.

12. Method according to claim 1, **characterised in** detecting the fluorescence signal of the interacted fluorochromatic reagent.

13. Method according to claim 1, **characterised in** detecting the fluorescence signal by using spectrophotometry or spectrofluorometry.

14. Use of method according to any of claims 1 - 13 for monitoring the amount of transparent exopolymer particles in water-intensive processes, such as water purification processes or water-intensive manufacturing processes.

15. Use according to claim 14, **characterised in that** the process includes at least one microfiltration, ultrafiltration, nanofiltration and/or reverse osmosis step(s).

## Patentansprüche

1. Verfahren zum Detektieren von transparenten Exopolymerpartikeln in einer Wasserprobe, wobei das Verfahren umfasst:
- Erhalten einer Wasserprobe,
- Einführen eines fluorochromatischen Reagenzes in die Wasserprobe, wobei das fluorochromatische Reagenz für vicinale Hydroxidgruppen von transparenten Exopolymerpartikeln spezifisch ist, wodurch sich das Fluoreszenzsignal des Reagenzes ändert, wenn es mit transparenten Exopolymerpartikeln in Kontakt kommt,
- Detektieren des Fluoreszenzsignals von der Wasserprobe und Bestimmen des Levels an transparentem Exopolymerpartikel der Probe.

2. Verfahren gemäß Anspruch 1, **dadurch gekennzeichnet, dass** das fluorochromatische Reagenz ein Boronsäurederivat, zum Beispiel 8-Chinolinboronsäure, 5-Chinolinboronsäure, 6-(Dimethylamino)-naphthalin-2-boronsäure oder Phenoxathiin-4-boronsäure, ist.

3. Verfahren gemäß Anspruch 1, **dadurch gekennzeichnet, dass** das fluorochromatische Reagenz ein Phenylboronsäurederivat, zum Beispiel 3-(Dansylamino)phenylboronsäure, 3,4,5-Trifluorphenylboronsäure, 2-Fluor-5-nitrophenylboronsäure, 2-Methoxyphenylboronsäure, N-Benzyl-3-pyridiniumphenylboronsäure, o-Dimethylaminomethylphenylboronsäure, 3-Chlor-4-fluorphenylboronsäure oder 4-Bromphenylboronsäure, vorzugsweise 3-(Dansylamino)phenylboronsäure, ist.

4. Verfahren gemäß Anspruch 1, **dadurch gekennzeichnet, dass** die Wasserprobe aus einem Wasserbehandlungsverfahren erhalten wird und die Zugabe einer oder mehrerer Verfahrenschemikalie(n) auf der Basis des detektierten Fluoreszenzsignals eingestellt und/oder ausgewählt wird.

5. Verfahren gemäß einem der vorangehenden Ansprüche 1 bis 4, **dadurch gekennzeichnet, dass** der pH der Wasserprobe auf einen konstanten Wert eingestellt wird, bevor das fluorochromatische Reagenz eingeführt wird.

6. Verfahren gemäß Anspruch 5, **dadurch gekennzeichnet, dass** das fluorochromatische Reagenz ein Phenylboronsäurederivat, zum Beispiel 3-(Dansylamino)phenylboronsäure, ist und der pH der Wasserprobe auf einen Level > 7, mehr bevorzugt > 8, eingestellt wird.

7. Verfahren gemäß Anspruch 5, **dadurch gekennzeichnet, dass** das fluorochromatische Reagenz 8-Chinolinboronsäure ist und der pH der Wasserprobe auf pH 4-10, mehr bevorzugt pH 4,5-7,5, eingestellt wird.

8. Verfahren gemäß Anspruch 5, **dadurch gekennzeichnet, dass** das fluorochromatische Reagenz 5-Chinolinboronsäure ist und der pH der Wasserprobe auf pH > 4, mehr bevorzugt pH > 7,5, eingestellt wird.

9. Verfahren gemäß Anspruch 5, **dadurch gekennzeichnet, dass** das fluorochromatische Reagenz Phenoxathiin-4-boronsäure ist und der pH der Wasserprobe auf pH 2-7, mehr bevorzugt pH 2-4, eingestellt wird.

10. Verfahren gemäß einem der vorangehenden Ansprüche 1-9, **dadurch gekennzeichnet, dass**
- die Wasserprobe als ein Nebenstrom von einem wässrigen Prozessstrom erhalten wird,
- das fluorochromatische Reagenz in den Nebenstrom eingeführt wird und das Fluoreszenzsignal detektiert wird,
- das Signal gegebenenfalls gefiltert wird,
- der Level an transparentem Exopolymerpartikel in dem Verfahrensstrom durch Vergleichen des detektierten Signals mit (einem) vorbestimmten Referenzsignal(en) bestimmt wird.

11. Verfahren gemäß Anspruch 1, **dadurch gekennzeichnet, dass** das Fluoreszenzsignal des freien, nicht-interagierten fluorochromatischen Reagenzes detektiert wird.

12. Verfahren gemäß Anspruch 1, **dadurch gekennzeichnet, dass** das Fluoreszenzsignal des interagierten fluorochromatischen Reagenzes detektiert wird.

13. Verfahren gemäß Anspruch 1, **dadurch gekennzeichnet, dass** das Fluoreszenzsignal unter Verwendung von Spektrophotometrie oder Spektrofluorometrie detektiert wird.

14. Verwendung des Verfahrens gemäß einem der Ansprüche 1 bis 13, um die Menge an transparenten Exopolymerpartikeln bei wasserintensiven Verfahren, zum Beispiel Wasserreinigungsverfahren oder wasserintensiven Herstellungsverfahren, zu überwachen.

15. Verwendung gemäß Anspruch 14, **dadurch gekennzeichnet, dass** das Verfahren wenigstens (einen) Mikrofiltrations-, Ultrafiltrations-, Nanofiltrations- und/oder Reverse-Osmose-Schritt(e) umfasst.

## Revendications

1. Procédé de détection de particules d'exopolymère transparentes dans un échantillon d'eau, le procédé comprenant
- l'obtention d'un échantillon d'eau,
- l'introduction d'un réactif fluorochromatique dans l'échantillon d'eau, le réactif fluorochromatique étant spécifique de groupes hydroxyde vicinaux de particules d'exopolymère transparentes, moyennant quoi le signal de fluorescence du réactif change lorsqu'il vient en contact avec des particules d'exopolymère transparentes,
- la détection du signal de fluorescence provenant de l'échantillon d'eau et la détermination du taux de particules d'exopolymère transparentes de l'échantillon.

2. Procédé selon la revendication 1, **caractérisé en ce que** le réactif fluorochromatique est un dérivé d'acide boronique, tel que l'acide 8-quinoléineboronique ; l'acide 5-quinoléineboronique ; l'acide 6-(diméthylamino)-naphtalène-2-boronique ; ou l'acide phénoxathiine-4-boronique.

3. Procédé selon la revendication 1, **caractérisé en ce que** le réactif fluorochromatique est un dérivé d'acide phénylboronique, tel que l'acide 3-(dansylamino)phénylboronique, l'acide 3,4,5-trifluorophénylboronique, l'acide 2-fluoro-5-nitrophénylboronique, l'acide 2-méthoxyphénylboronique, l'acide N-benzyl-3-pyridiniumphénylboronique, l'acide o-diméthylaminométhylphénylboronique, l'acide 3-chloro-4-fluorophénylboronique ou l'acide 4-bromophénylboronique, de préférence l'acide 3-(dansylamino)phénylboronique.

4. Procédé selon la revendication 1, **caractérisé par** l'obtention de l'échantillon d'eau à partir d'un processus de traitement de l'eau, et l'ajustement et/ou la sélection de l'alimentation d'un ou plusieurs produits chimiques de processus sur la base du signal de fluorescence détecté.

5. Procédé selon l'une quelconque des revendications 1 à 4 précédentes, **caractérisé par** l'ajustement du pH de l'échantillon d'eau à une valeur constante avant introduction du réactif fluorochromatique.

6. Procédé selon la revendication 5, **caractérisé en ce que** le réactif fluorochromatique est un dérivé d'acide phénylboronique, tel que l'acide 3-(dansylamino)phénylboronique, et le pH de l'échantillon d'eau est ajusté à un niveau > 7, de manière davantage préférée > 8.

7. Procédé selon la revendication 5, **caractérisé en ce que** le réactif fluorochromatique est l'acide 8-quinoléineboronique et le pH de l'échantillon d'eau est ajusté à pH 4 à 10, de manière davantage préférée pH 4,5 à 7, 5.

8. Procédé selon la revendication 5, **caractérisé en ce que** le réactif fluorochromatique est l'acide 5-quinoléineboronique et le pH de l'échantillon d'eau est ajusté à pH > 4, de manière davantage préférée pH > 7,5.

9. Procédé selon la revendication 5, **caractérisé en ce que** le réactif fluorochromatique est l'acide phénoxathiin-4-boronique et le pH de l'échantillon d'eau est ajusté à pH 2 à 7, de manière davantage préférée pH 2 à 4.

10. Procédé selon l'une quelconque des revendications 1 à 9, **caractérisé par**
- l'obtention de l'échantillon d'eau en tant que courant secondaire issu d'un courant de processus aqueux,
- l'introduction du réactif fluorochromatique dans le courant secondaire et la détection du signal de fluorescence,
- facultativement le filtrage du signal,
- la détermination du taux de particules d'exopolymère transparentes dans le courant du processus par comparaison du signal détecté à un (des) signal (signaux) de référence prédéterminé(s).

11. Procédé selon la revendication 1, **caractérisé par** la détection du signal de fluorescence du réactif fluorochromatique libre n'ayant pas interagi.

12. Procédé selon la revendication 1, **caractérisé par** la détection du signal de fluorescence du réactif fluorochromatique ayant interagi.

13. Procédé selon la revendication 1, **caractérisé par** la détection du signal de fluorescence par l'utilisation d'une spectrophotométrie ou une spectrofluorométrie.

14. Utilisation d'un procédé selon l'une quelconque des revendications 1 à 13, pour contrôler la quantité de particules d'exopolymère transparentes dans des processus gourmands en eau, tels que des processus de purification d'eau ou des processus de fabrication gourmands en eau.

15. Utilisation selon la revendication 14, **caractérisée en ce que** le processus inclut au moins une étape de microfiltration, ultrafiltration, nanofiltration et/ou osmose inverse.
